(19) Europäisches Patentamt
European Patent Office
Office européen des brevets

(11) **EP 2 365 338 A1**

(12) **EUROPEAN PATENT APPLICATION**

(43) Date of publication:
**14.09.2011 Bulletin 2011/37**

(51) Int Cl.:
***G01N 33/84*** (2006.01)

(21) Application number: **11157639.3**

(22) Date of filing: **10.03.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **10.03.2010 JP 2010053214**

(71) Applicants:
• **Fujifilm Corporation**
**Minato-ku**
**Tokyo (JP)**
• **National University Corporation**
**Hokkaido University**
**Sapporo-shi, Hokkaido 060-0808 (JP)**

(72) Inventors:
• **Adachi, Takashi**
**Ashigarakami-gun**
**Kanagawa-ken (JP)**
• **Ogikubo, Shinya**
**Ashigarakami-gun**
**Kanagawa-ken (JP)**
• **Ohta, Nobuhiro**
**Sapporo-shi**
**Hokkaido (JP)**
• **Nakabayashi, Takakazu**
**Sapporo-shi**
**Hokkaido (JP)**

(74) Representative: **HOFFMANN EITLE**
**Patent- und Rechtsanwälte**
**Arabellastraße 4**
**81925 München (DE)**

(54) **Low-oxygen-region-analysis method and apparatus by time-resolved-measurement of light-induced-autofluorescence from biological-sample**

(57)    It is possible to highly accurately measure the oxygen concentration of a substance to be measured without being influenced by the intensity of excitation light (L1), the concentration of fluorescent material, and the surrounding environment. Pulsed excitation light (L1) including a wavelength that can excite a fluorescent material contained in living matter is generated. The fluorescence lifetime (Tf) of the fluorescent material is longer than or equal to 4.8 nanoseconds. A predetermined position in the living matter is illuminated with the pulsed excitation light (L1). Further, light including fluorescence (Lf) emitted from the fluorescent material excited by illumination with the pulsed excitation light (L1) is received. The lifetime (Tf) of the fluorescence (Lf) included in the received light is calculated by time-resolving the intensity of the fluorescence (Lf) . Further, the oxygen concentration of the living matter is measured based on the lifetime (Tf)

FIG.1

**Description**

Field of the Invention

The present invention relates to a method for measuring the concentration of oxygen of living matter, a method for detecting a low oxygen concentration region based on the concentration of oxygen, a method for analyzing the living matter based on the result of measurement and the result of detection, and apparatuses for carrying out these methods.

Description of the Related Art

As a method for distinguishing normal tissue and abnormal tissue of living matter from each other, various methods have been tried. In the methods, the oxygen concentration of the living matter is measured based on a difference in the physical properties of a substance that is used as a label, and the normal tissue and the abnormal tissue are identified based on the difference in oxygen concentration.

For example, there is a method in which a fluorescent material that is present in living matter is used as a label, and a difference in the intensity of fluorescence (autofluorescence) emitted from the fluorescent material by excitation and a fluorescence decay spectrum are detected. PCT Japanese Publication No. 2001-509589 (Patent Document 1) discloses a laser-induced fluorescence attenuation spectroscopy method (LIFAS). In the LIFAS, the oxygen concentration of living matter in a sample is measured by detecting a difference in the intensity of autofluorescence emitted from the living matter sample and the radiation characteristic of light, such as a decay spectrum. Further, a change in the pathological condition, such as malignant tumor condition, is detected.

In this method, it is not necessary to label the living matter with a harmful substance, such as a fluorescent dye. Therefore, it is possible to measure the oxygen concentration and to identify normal tissue and abnormal tissue without greatly damaging the target of measurement.

Further, Japanese Unexamined Patent Publication No. 2006-282653 (Patent Document 2) or the like discloses a method using, as a label, a probe for image diagnosis (diagnosis using images) that exhibits a different activation level depending on the oxygen concentration of living matter during administration. In the method, a difference in the activation level is detected and measured to detect a change in the physiological condition or the pathological condition of the living matter. In this method, since the probe that is a drug is directly administered, high sensitivity detection is possible.

However, in the method based on the fluorescence intensity and the fluorescence decay spectrum, since the fluorescence intensity and the fluorescence decay spectrum differ depending on a region to be detected and each individual, it is necessary to obtain reference for each detection region and individual. Further, the amount of fluorescence emitted from a fluorescent material changes depending on the concentration of the fluorescent material, the intensity of excitation light, and the like. Therefore, in the method disclosed in Patent Document 1, excellent quantitative detection and high sensitivity detection are difficult, and the method is not sufficiently reliable.

Further, in the method disclosed in Patent document 2, since the probe is directly administered, there is a risk of greatly damaging the target of measurement. Further, it needs time to lead the probe to a target region and to activate the probe. Hence, there is a problem that quick measurement is not possible.

SUMMARY OF THE INVENTION

In view of the foregoing circumstances, it is an object of the present invention to provide a measurement method for measuring the concentration of oxygen of living matter quickly and at high sensitivity without greatly damaging a target of measurement. Further, it is another object of the present invention to provide a detection method for detecting a low oxygen region based on the oxygen concentration. Further, it is still another object of the present invention to provide a method for analyzing the living matter based on the oxygen concentration and the low oxygen region.

Further, it is another object of the present invention to provide apparatuses for carrying out the measurement method, the detection method, and the method for analyzing the living matter.

A measurement method according to the present invention is a measurement method comprising the steps of:

generating pulsed excitation light including a wavelength that can excite a fluorescent material contained in living matter, the fluorescence lifetime of the fluorescent material being longer than or equal to 4.8 nanoseconds;
illuminating a predetermined position in the living matter with the pulsed excitation light;
receiving light including fluorescence emitted from the fluorescent material excited by illumination with the pulsed excitation light;
calculating the lifetime (fluorescence lifetime) of the fluorescence included in the received light by time-resolving the intensity of the fluorescence; and
measuring the oxygen concentration of the living matter based on the lifetime.

A measurement apparatus according to the present invention is a measurement apparatus comprising:

an excitation light generation means that generates pulsed excitation light including a wavelength that can excite a fluorescent material contained in living matter, the fluorescence lifetime of the fluorescent material being longer than or equal to 4.8 nanoseconds;

an excitation light illumination means that illuminates a predetermined position in the living matter with the pulsed excitation light;

a light receiving means that receives light including fluorescence emitted from the fluorescent material excited by illumination with the pulsed excitation light;

a time-resolving means that time-resolves fluorescence of a predetermined wavelength included the fluorescence included in the received light synchronously with illumination with the pulsed excitation light;

a detection means that detects the time-resolved fluorescence; and

a measurement means that calculates the lifetime of the fluorescence emitted from the fluorescent material based on the time-resolved fluorescence detected by the detection means, and measures the oxygen concentration of the living matter based on the lifetime.

Here, the expression "a fluorescent material contained in living matter, the fluorescence lifetime of the fluorescent material being longer than or equal to 4.8 nanoseconds" refers to a fluorescent material the lifetime of which is longer than or equal to 4.8 nanoseconds in living matter of normal condition. The fluorescent material may be in any state, such as a protein bond state and a free state, or an average value of a region in a tissue or a cell. Further, the expression "in living matter" means "in an environment with a temperature of 37°C". Therefore, the value is valuable by some difference in temperature or the like. Further, the term "living matter" refers to a cell or a tissue of a human or an animal, a biological tissue, such as body fluid, and the like. The living matter may be any kind of matter including matter extracted from an organism, cultured matter, a cell or tissue in vivo, and the like.

In the measurement method and the measurement apparatus of the present invention, it is desirable that the fluorescent material is at least one kind of fluorescent material selected from the group consisting of porphyrins, flavin enzymes, collagen, and elastin. Especially, it is desirable that the fluorescence lifetime of the fluorescent material is longer than or equal to 13.3 nanoseconds.

In the measurement method of the present invention, it is desirable that the lifetime of the fluorescence included in the received light is calculated for each wavelength by wavelength-resolving the fluorescence to obtain fluorescence spectrum of the fluorescence and by time-resolving, based on the fluorescence spectrum, the intensity of the fluorescence for the respective wavelengths. Therefore, it is desirable that the measurement apparatus of the present invention includes a light receiving means for obtaining such a spectrum.

In the measurement method and the measurement apparatus of the present invention, the predetermined position may be a plurality of positions. When the measurement apparatus that is structured as described above includes a low oxygen region detection means for detecting a low oxygen region in the living matter, it is possible to detect the low oxygen region in the living matter.

In detection of the low oxygen region, the low oxygen region in the living matter may be detected by generating and displaying an image of the low oxygen region.

Further, the living matter may be analyzed by identifying the pathological condition of the living matter, such as presence of malignant tumor condition, based on the oxygen concentration measured by using the measurement method of the present invention and the low oxygen region detected by using the detection method of the present invention.

Optionally, a measurement apparatus according to the present invention may be a microscope further including:

a stage that keeps the living matter in contact with a surface of the stage, and which is movable in three-dimensional directions so that an arbitrary position in the living matter is illuminated with the pulsed excitation light; and

a position adjustment means that moves the stage to an arbitrary position in three-dimensional directions,

wherein the excitation light illumination means includes an optical system that receives the pulsed excitation light and illuminates the living matter with the pulsed excitation light, and

wherein the light receiving means includes an optical system that receives the light including the fluorescence emitted from the fluorescent material excited by illumination with the pulsed excitation light, and that guides the light to the time-resolving means.

Optionally, a measurement apparatus of the present invention may be a measurement apparatus further including:

a stage that keeps the living matter in contact with a surface of the stage, and which is movable so that an arbitrary position in the living matter, at least in the direction of an optical axis of the pulsed excitation light, is illuminated with the pulsed excitation light;

a first position adjustment means that moves the stage to an arbitrary position at least in the direction of the optical axis; and

a second position adjustment means that moves the excitation light illumination means so that an arbitrary position at least in an in-plane direction, which is perpendicular to the optical axis of the pulsed excitation light, in the living matter is illuminated with the pulsed excitation light,

wherein the excitation light illumination means includes an optical system that receives the pulsed excitation light and illuminates the living matter with the pulsed excitation light, and

wherein the light receiving means includes an optical system that receives the light including the fluorescence emitted from the fluorescent material excited by illumination with the pulsed excitation, light, and that guides the light to the time-resolving means.

Optionally, a measurement apparatus of the present invention may be a measurement apparatus further including:

a position adjustment means that moves the excitation light illumination means so that an arbitrary position in three-dimensional directions in the living matter is illuminated with the pulsed excitation light,

wherein the excitation light illumination means includes an optical system that receives the pulsed excitation light and illuminates the living matter with the pulsed excitation light, and

wherein the light receiving means includes an optical system that receives the light including the fluorescence emitted from the fluorescent material excited by illumination with the pulsed excitation light, and that guides the light to the time-resolving means.

Optionally, a measurement apparatus of the present invention may be a measurement apparatus, wherein the excitation light illumination means includes at least one optical fiber for illumination that illuminates the living matter with the pulsed excitation light, and

wherein the light receiving means includes at least one optical fiber for receiving light that receives the light including the fluorescence emitted from the fluorescent material excited by illumination with the excitation light and that guides the fluorescence to the time-resolving means. When the measurement apparatus of the present invention is structured in such a manner, it is desirable that the at least one optical fiber for illumination and the at least one optical fiber for receiving light form a bundle fiber. Further, it is more desirable that the bundle fiber is formed by bundling an optical fiber for illumination and a plurality of optical fibers for receiving light together in such a manner that the outer surface of the optical fiber for illumination arranged substantially at the center of the bundle fiber is surrounded by the plurality of optical fibers for receiving light.

The inventors of the present invention noted that the fluorescence lifetime, which is a unique value to each fluorescent material, may be used to highly accurately measure the oxygen concentration without being influenced by the intensity of excitation light, the concentration of the fluorescent material, fading of the fluorescent material, and fluorescence from unwanted substance other than desirable fluorescence. It is known that the oxygen concentration of an abnormal cell, such as a cancer cell, is lower than the oxygen concentration of a normal cell.

Measurement of oxygen concentration based on fluorescence lifetime is reported also in "Time-resolved optical imaging provides a molecular snapshot of altered metabolic function in living human cancer cell models". D. Sud et al., OPTICS EXPRESS, Vol. 14, No. 10, pp. 4412-4426, 2006 (Non-Patent Document). The non-patent document tries to detect a difference in the oxygen concentration of living matter based on the fluorescence lifetime of NADH (nicotinamide adenine dinucleotide), which is an autofluorescent material in living matter, and to distinguish a cancer cell and a normal cell from each other. The non-patent document describes that the fluorescence lifetime of a normal cell is longer than the fluorescence lifetime of an abnormal cell. However, it is supposed that the fluorescence lifetime of a normal cell is shorter than the fluorescence lifetime of an abnormal cell, because the oxygen concentration of the normal cell is fundamentally higher than the oxygen concentration of the abnormal cell. Therefore, it is impossible to recognize that the non-patent document detects a change in the oxygen concentration.

In living matter, the amount of change in oxygen concentration by abnormality is, for example, in the range of from 15 to 40 mmHg in a normal tissue, and in the range of from 0 to 10 mmHg in a cancerous tissue. Therefore, the difference is extremely small, and it is difficult to accurately detect the difference in oxygen concentration, as a difference in fluorescence lifetime. Hence, it is most likely that the result, as described in the non-patent document, in which the fluorescence lifetime of the normal cell is longer than that of the abnormal cell, is obtained in some cases. With such accuracy level of detection, it is impossible to detect oxygen concentration based on a correlation between the amount of change in oxygen concentration and fluorescence lifetime.

The inventors of the present invention intensively studied the reasons for the low accuracy of measurement. They noted that the amount of change in the fluorescence lifetime of an autofluorescent material, which is a target of measurement, with respect to oxygen concentration varies depending on the value of fluorescence lifetime, and discovered a fluorescence lifetime value that can make highly accurate measurement possible. Specifically, the fluorescence lifetime of hematoporphyrinwas measured many times by using a time-correlated single photon counting method (TCSPC) and by using a time gate method, and a marginal value that can separate two fluorescence lifetimes in the current fluorescence lifetime measurement technique was discovered. Further, the inventors of the present invention discovered a fluorescence lifetime value for accurately measuring oxygen concentration. The details about the discovered fluorescence lifetime value will be described later. It was not easy to find the fluorescence lifetime value for accurately measuring oxygen concentration, even if the disclosure in the non-patent document and the common technical knowledge at the time of study were taken into consideration.

The oxygen concentration measurement method of the present invention is based on the findings by the inventors of the present invention that when the oxygen concentration of living matter is measured based on the fluorescence lifetime of an autofluorescent material contained in the living matter, if a fluorescent material the fluorescence lifetime of which is longer than or equal to 4.8 nanosecond is adopted as the autofluorescent material, highly accurate detection is realized. Since the fluorescence lifetime is a value unique to each fluorescent material, it is possible to highly accurately measure the oxygen concentration of living matter by using the method of the present invention without being influenced by the intensity of excitation light, the concentration of the fluorescent material, and fluorescence from unwanted substance.

Further, in the present invention, the fluorescence lifetime of an autofluorescent material is a target of measurement. Therefore, administration of drug or the like to the living matter is not necessary. Hence, it is possible to measure the oxygen concentration without greatly damaging the living matter.

Highly accurate measurement of oxygen concentration is possible in the present invention. Therefore, it is possible to identify not only the normality/abnormality of pathological condition, but the degree of abnormality. Especially when the efficacy of treatment of a disease is dependent on oxygen concentration, it is possible to provide data for appropriately judging treatment policy, such as the dose of medicine to be administered and the intensity of physical therapy.

BRIEF DESCRIPTION OF THE DRAWINGS

Figure 1 is a schematic diagram illustrating an oxygen concentration measurement apparatus, a low oxygen region detection apparatus and a living matter analysis apparatus according to a first embodiment of the present invention;
Figure 2 is a diagram illustrating a relationship between oxygen concentration and fluorescence lifetime;
Figure 3A is a diagram illustrating excitation light absorption characteristics of major autofluorescent materials contained in living matter:
Figure 3B is a diagram illustrating fluorescence spectra of the autofluorescent materials illustrated in Figure 3A;
Figure 4 is a diagram illustrating a method for calculating fluorescence lifetime by resolving time;
Figure 5 is a schematic diagram illustrating the configuration of a wide-field-type oxygen concentration measurement apparatus, a low oxygen region detection apparatus and a living matter analysis apparatus;
Figure 6 is a schematic diagram illustrating the configuration of a confocal-type oxygen concentration measurement apparatus, a low oxygen region detection apparatus and a living matter analysis apparatus;
Figure 7 is is a schematic diagram illustrating the configuration of an oxygen concentration measurement apparatus, a low oxygen concentration, detection apparatus, and a living matter analysis apparatus according to a second embodiment of the present invention;
Figure 8A is a schematic diagram illustrating the structure of an endoscope when a fiber bundle is provided in the endoscope; and
Figure 8B is a schematic diagram illustrating the structure of an endoscope when a fiber probe is provided in the endoscope.

DESCRIPTION OF THE PREFERRED EMBODIMENTS

"First Embodiment of Oxygen Concentration Measurement Apparatus (Measurement Apparatus) (Microscope)"

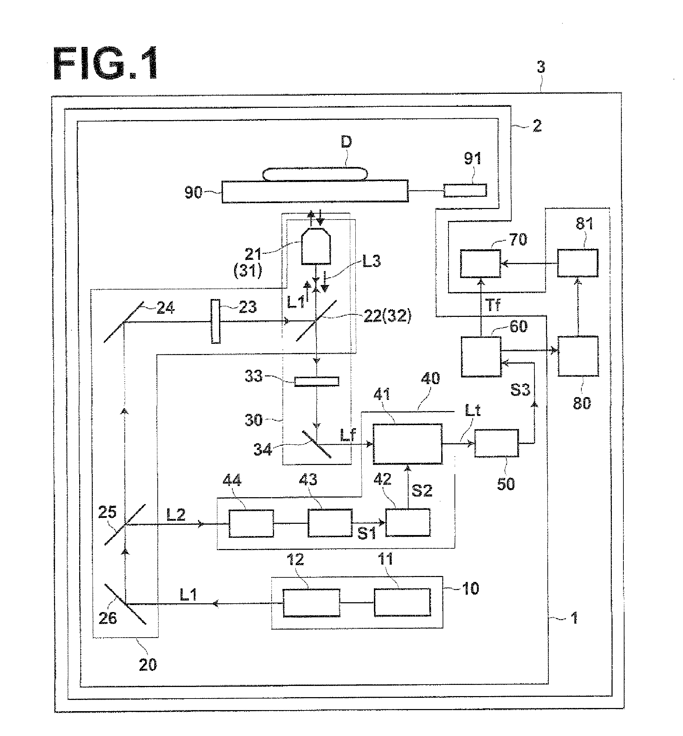

An oxygen concentration measurement apparatus and an oxygen concentration measurement method according to a first embodiment of the present invention will be described with reference to drawings. Figure 1 is a schematic diagram illustrating the configuration of an oxygen concentration measurement apparatus 1 according to an embodiment of the present invention. In Figure 1, each unit is illustrated at an appropriate scale so as to be easily recognized.

As illustrated in Figure 1, the oxygen concentration measurement apparatus 1 outputs pulsed excitation light L1 (hereinafter, referred to as excitation light L1) to substance D to be measured, which is living matter, by an excitation light illumination means 20. Further, the oxygen concentration measurement apparatus 1 obtains fluorescence by receiving light L3 by a light receiving means 30. The light L3 includes fluorescence Lf emitted, by illumination with the excitation light L1, from fluorescent material P contained in the substance D to be measured. Further, a time-resolving means 40 time-resolves the fluorescence into predetermined fluorescence Lf and a detection means 50 detects the time-resolved fluorescence Lf. Further, a measurement means 60 obtains a fluorescence decay curve based on the intensity of the detected fluorescence Lf, and calculates lifetime Tf of the fluorescence Lf. Further, the oxygen concentration of the substance D to be measured is measured based on the lifetime Tf. The excitation light L1 includes a wavelength that can excite fluorescent material P contained in the substance D to be measured, the lifetime of the fluorescent material P being longer than or equal to 4.8 nanoseconds.

The method for measuring the fluorescence lifetime is basically divided into a time domain measurement method and a frequency domain measurement method. In the present embodiment, the time domain measurement method is adopted. Examples of the time domain measurement method are a streak camera method, a time correlated single photon counting method, a time gate method, and the like. In the present embodiment, a case of measuring fluorescence lifetime by using the time gate method will be described.

In the time gate method, emitted fluorescence is divided to a certain time domain synchronously with illumination with pulsed excitation light. Further, the integral value of fluorescence intensity in the range of the time domain is detected by a solid-state detector, such as a CCD, and measurement is performed for a different time domain. Accordingly, a fluorescence decay curve is approximated In the present embodiment, when signal light L2 separated from the excitation light L1 by a beam splitter 25 is detected by a photodiode 44, a synchronization control apparatus 43 sends synchronization signal S1 to a gate controller 42. When the gate controller 42 receives the synchronization signal S1, the gate controller 42 originates gate signal S2 in a predetermined time domain that has been set in advance, and an image intensifier 41 with a gate function is driven. Fluorescent image Lf that has been input to the image intensifier 41 with a gate function is intensified in a time domain corresponding to the gate signal S2. Further, the detection means 50 detects the integral value (fluorescent signal) Lt of the fluorescence intensity of the predetermined fluorescence Lf. This process is repeated based on the gate signal S2. Further, the measurement means 60 calculates fluorescence lifetime Tf by performing analysis by a single exponential function or the like.

The inventors of the present invention measured the fluorescence lifetime of hematoporphyrin many times by using a time-correlated single photon counting method (TCSPC) and by using a time gate method, and discovered that a marginal value that can separate two fluorescence lifetimes in the current fluorescence lifetime measurement technique is 0.03 nsec. Further, they discovered the fluorescence lifetime value of a material that can exhibit such a difference in fluorescence lifetime.

Oxygen concentration and fluorescence lifetime have the following relationship by Stern-Volmer equation:

$$1/\tau = 1/\tau 0 + kq * [\text{Oxygen Concentration}],$$

where
$\tau$ : fluorescence lifetime at certain oxygen concentration,
$\tau 0$: fluorescence lifetime when the oxygen concentration is 0, and
kq: quenching constant.

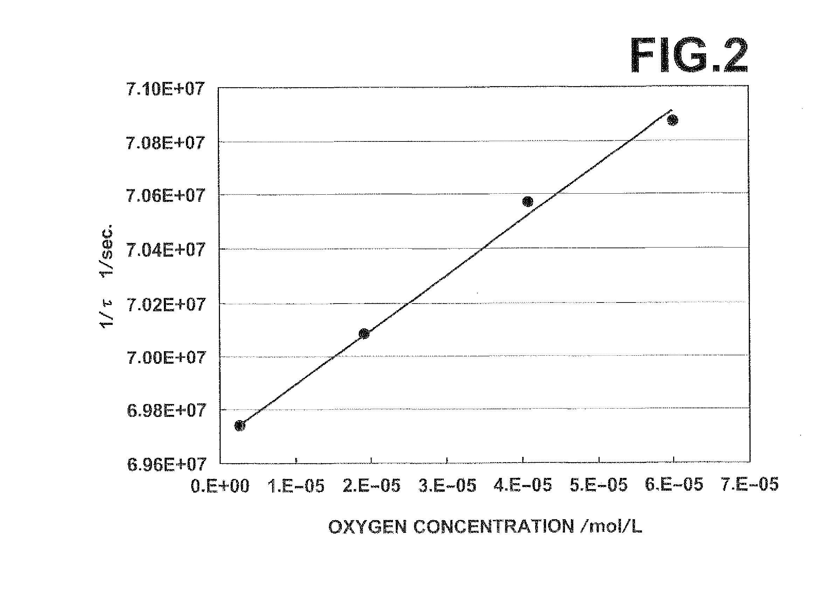

A hematoporphyrin aqueous solution was used to obtain the value of quenching constant kq to be applied to living matter. The oxygen concentration of the solution was changed, by oxygen bubbling, in a low concentration range appropriate for living matter. While the oxygen concentration of the solution was changed, the fluorescence lifetime was measured by using a single photon counting method (measurement apparatus: a measurement system including Time-to-Amplitude Converter (TAC; ORTEC457) and the like) . The oxygen concentration was measured by using an optical oxygen concentration meter (*Precision Sensing Co.: 1-channel-type high-sensitivity micro oxygen meter, Microx TX3-trace) . The wavelength of excitation light was 420 nm, and fluorescence with the wavelength of 615 nm was detected. The result of detection was plotted to obtain Stern-Volmer plot (Figure 2), and fitting was performed on the Stern-Volmer plot by using a least square method. Consequently, the following result was obtained:

$$1/\tau = 1/(14.35 \times 10^{-9}) + 20.01 \times 10^{9} \times [\text{Oxygen Concentration}].$$

Further, quenching constant kq and fluorescence lifetime $\tau 0$ were obtained as follows:

$$kq=20.01\times10^{9}\ L/(mol\cdot sec);$$

and $\tau 0$=14.35 nsec. The correlation coefficient was 0.9975.

The obtained quenching constant kq was applied to living matter, and a difference in fluorescence lifetime between normal tissue and abnormal tissue was obtained. When the fluorescence lifetime in normal cell is $\tau 1$, and the fluorescence lifetime in abnormal cell is $\tau 2$, the value of ($\tau 2$-$\tau 1$) was estimated by using Stern-Volmer equation using the quenching constant kq=20.01$\times10^{9}$L/(mol·sec).

The value of $\tau 0$ was estimated from $1/\tau 1=1/\tau 0+20.01\times10^{9}$*[Oxygen Concentration of Normal Tissue]. Further, the value of $\tau 2$ was estimated from $1/\tau 2=1/\tau 0+20.01\times10^{9}$* [Oxygen Concentration of Abnormal Tissue].

The oxygen concentration at the temperature of 37°C is represented as follows:

$$[Oxygen\ Concentration]\ =\ [Partial\ Pressure\ of\ Oxygen\ mmHg]/760\ mmHg\ \times\ [Solubility\ Coefficient]/22.4(L/mol).$$

When the temperature is 37°C, the solubility coefficient is 0.024. When the partial pressure of oxygen of normal tissue is in the range of from 15 to 40 mmHg, the oxygen concentration is in the range of from $2.1\times10^{-5}$ to $5.6\times10^{-5}$ mol/L. Further, when the partial pressure of oxgen of abnormal tissue is in the range of from 0 to 10 mmHg, the oxygen concentration is in the range of from 0 to $1.4\times10^{-5}$ mol/L.

When the partial pressure of oxygen of the normal tissue is 40 mmHg and the partial pressure of oxygen of the abnormal tissue is 0 mmHg, a difference in the partial pressure is large. In that case, when $\tau 2$=4.8 nsec, $\tau 2$-$\tau 1$=0.03 nsec. Meanwhile, when the partial pressure of oxygen of the normal tissue is 15 mmHg and the partial pressure of oxygen of the abnormal tissue is 10 mmHg, a difference in the partial pressure is small. In that case, when $\tau 2$=13.3 nsec, $\tau 2$-$\tau 1$=0.03 nsec. Therefore, if $\tau 2$ is longer than or equal to 4.8 nsec, it is possible to distinguish and separate normal tissue and abnormal tissue from each other. Further, when $\tau 2$ is longer than or equal to 13.3 nsec, it is possible to highly accurately distinguish normal tissue and abnormal tissue from each other. The lowest value of partial pressure of oxygen in abnormal tissue maybe substantially zero in some cases. Therefore, the value of 0 mmHg was used as the lowest partial pressure for the abnormal tissue.

Therefore, in the embodiment of the present invention, it is desirable to use, as fluorescent material P (autofluorescent material) contained in substance D to be measured, a fluorescent material the fluorescence lifetime of which is longer than or equal to 4.8 nanoseconds. Optionally, a fluorescent material the fluorescence lifetime of which is longer than or equal to 13.3 nanoseconds may be used.

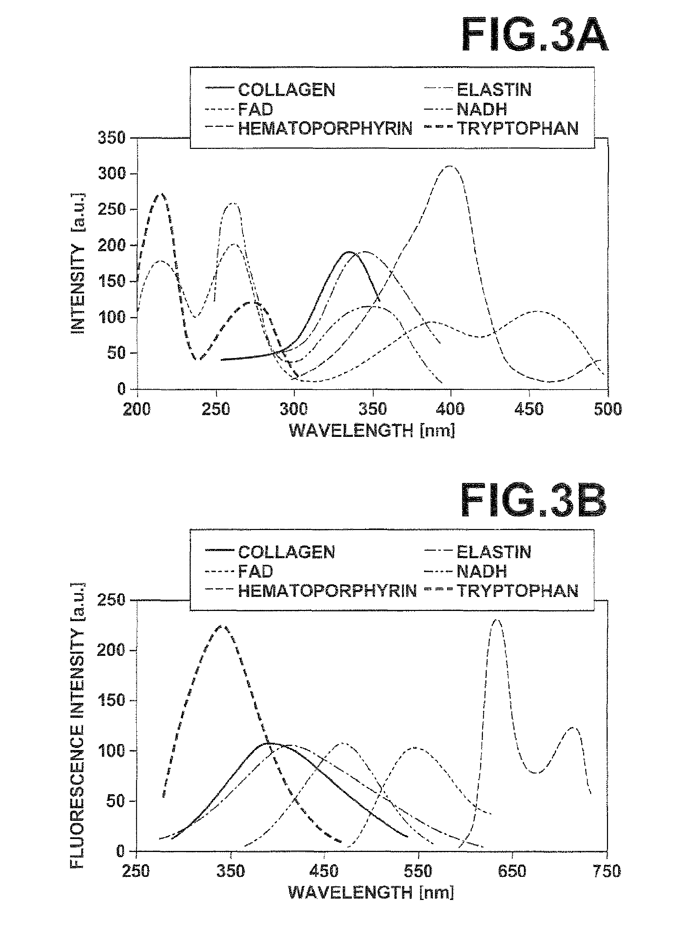

Table 1 shows major autofluorescent materials and fluorescence lifetime values of the autofluorescent materials measured by the inventors of the present invention by using a time-correlated single photon counting method (TCSPC) . Table 1 shows values when each of the autofluorescent materials are in a free state. Further, Figures 3A and 3B are diagrams illustrating excitation light absorption characteristics and fluorescence spectrum of major autoflucrescent materials.

[Table 1]

| Fluorescent Material in Biological Sample | Fluorescence Lifetime (ns) |
| --- | --- |
| Hematoporphyrin | 12.2 |
| Collagen | 5.1 |
| Elastin | 8.5 |
| Tryptophan | 2.8 |
| Pyridoxine | 0.85 |
| NADH (Nicotinamide Adenine Dinucleotide) | 0.4 |
| NADPH (Nicotinamide Adenine Dinucleotide Phosphate) | 0.4 |
| FAD (Flavin Adenine Dinucleotide) | 5.0 |

Since the oxygen concentration measurement apparatus 1 is a microscope, substance D to be measured is placed on a sample stage 90, which is movable in the directions of x, y and z by a position adjustment means 91. The position of the sample stage 90 is adjusted by the position adjustment means 91 to illuminate a predetermined position in the substance D to be measured with excitation light L1. The oxygen concentration measurement apparatus 1 may include a window (not illustrated) including a magnification lens or the like. The window is provided to check, from the upper side of the substance D to be measured, the illumination position of the excitation light L1 on the substance D to be measured, thereby a predetermined position is accurately illuminated with the excitation light L1.

In the oxygen concentration measurement apparatus 1, an excitation light generation means 10 includes a pulse laser 11 and a pulse picker 12. The pulse laser 11 can oscillate pulses including a wavelength that can excite fluorescent material P contained in the substance D to be measured, the fluorescent material P having a fluorescence lifetime longer than or equal to 4.8 nanosecond. The pulse picker 12 picks (thins) pulses oscillated by the pulse laser 11 so that pulses are output with predetermined intervals. The pulse laser 11 is not particularly limited, but desirably a laser that can oscillate ultra-short pulsed light. It is more desirable that the pulse laser 11 can oscillate pulses in the range of from some tens of femtoseconds to some tens of picoseconds. For example, the pulse laser 11 that has the wavelength of 420 nm, a pulse width of 3 ps, a repetition frequency of 80 MHz, a peak power of 8 kW, an average power of 2 W, and the like may be used. The wavelength of 420 nm is a second harmonics (SHG: second harmonic generation) of a titanium sapphire laser having an oscillation wavelength of 840 nm. Further, the excitation light generation means 10 may include a third harmonic (THG: third harmonic generation) or a wavelength conversion element, such as an optical parametric oscillator (OPO).

The fluorescence Lf may be excited in the substance D to be measured by multi-photon excitation. In that case, a titanium sapphire laser having an oscillation wavelength of 840 nm, a pulse width of 100 fs, a repetition frequency of 80 MHz, a peak power of 100 kW, an average power of 0.8 W and the like are used as excitation light L1 having a wavelength and a pulse width that can induce multi-photon excitation. In multi-photon excitation, it is possible to excite only a desirable position. Therefore, it is possible to reduce fluorescence in the vicinity of the position, and to further reduce noise. Further, since it is possible to reduce absorption decay before the observation position, it is possible to efficiently illuminate the observation position with the excitation light L1. Further, it is possible to analyze at high spatial resolution.

The excitation light L1 output from the excitation light generation means 10 enters the excitation light illumination means 20. Further, the excitation light illumination means 20 illuminates a predetermined position in the substance D to be measured with the excitation light L1. The structure of the excitation light illumination means 20 is not particularly limited. However, in the excitation light illumination means 20 of the present embodiment, the same objective lens (optical system) 21 (31) is used to output the excitation light L1 and to receive light L3 including fluorescence Lf emitted from the substance D to be measured by excitation of the fluorescent material P. Therefore, the excitation light illumination means 20 includes the objective lens 21 and a dichroic mirror 22 (32). The objective lens 21 is arranged so that a predetermined position in the substance D to be measured is illuminated with the excitation light L1 and that the light L3 is receivable. The dichroic mirror 22 (32) makes the excitation light L1 enter the objective lens 21, and guides the light L3 to the time-resolving means 40. It is desirable that the material of the objective lens 21 has high transmittance (transmission) with respect to the excitation light L1 and fluorescence. Further, it is desirable that the amount of fluorescence emitted from the lens material is small.

In the present embodiment, the time-resolving means 40 is driven synchronously with illumination with the excitation light L1. Therefore, the excitation light illumination means 20 includes a beam splitter 25 and an excitation filter 23. The beam splitter 25 separates, from the excitation light L1, signal light L2 that originates synchronization signal S1. The excitation filter 23 filters the excitation light L1 so that light having an wavelength that can excite the fluorescent material P remains. The excitation light illumination means 20 may include a mirror (24, 26), a lens (not illustrated), and the like for guiding the excitation light L1 if necessary.

The light receiving means 30 is not particularly limited as long as it can receive the light L3 emitted from the substance D to be measured by illumination with the excitation light L1, and output intended fluorescence Lf to the time-resolving means 40. However, in the present embodiment, the light receiving means 30 includes an objective lens 31 (21), a dichroic mirror 32 (22), and a filter 33. The objective lens 31 (21) receives light L3 emitted from the substance D to be measured. The dichroic mirror 32 (22) guides the light L3 to the time-resolving means 40, and the filter 33 removes noise light, such as scattered light, from the light L3.

The objective lens (optical system) 31 and the dichroic mirror 32 are as described above. A filter 34, such as a band-pass filter or a short-pass filter, may be appropriately used based on the wavelength of fluorescence Lf to be detected. When plural kinds of fluorescence Lf need to be observed, plural band-pass filters that pass light of different wavelength bands may be used. Alternatively, a filter that has a variable passing wavelength band may be used.

The fluorescence Lf received by the light receiving means 30 is input to the time-resolving means 40. The time-resolving means 40 extracts fluorescent signal Lt in a predetermined time domain from the fluorescence Lf, and outputs the extracted fluorescent signal Lt to the detection means 50. Time-resolving of fluorescence starts from time t0 when the first synchronization signal S1 for output of the excitation light L1 is received. The synchronization signal S1 is generated at an optical detector 44 by converting, to an electrical signal, the signal light L2 separated from the excitation light L1 by the beam splitter 25. Further, the synchronization signal S1 is sent from a synchronization control apparatus 43 to a gate controller 42.

The fluorescence Lf received by the light receiving means 30 is input to the time-resolving means 40. The time-resolving means 40 extracts a fluorescent image in a predetermined time domain from predetermined fluorescence Lf, and outputs the extracted fluorescent image to the detection means 50. Time-resolving of fluorescence starts from time t0 when the first synchronization signal S1 for output of the excitation light L1 is received. The synchronization signal S1 is generated at an optical detector 44 by converting, to an electrical signal, the signal light L2 separated from the excitation light L1 by the beam splitter 25. Further, the synchronization signal S1 is sent from a synchronization control apparatus 43 to a gate controller 42.

The light detector 44 is not particularly limited. However, it is desirable to use a photodiode or the like, which is generally used.

The synchronization control apparatus 43 is not particularly limited as long as it can receive a signal output from the light detector 44 and output synchronization signal S1 to the gate controller 42. For example, a personal computer (PC) or the like may be used as the synchronization control apparatus 43. The gate controller 42 receives the synchronization signal S1 and originates gate signal S2 in a predetermined time domain that has been set in advance. Accordingly, the gate controller 42 instructs opening/closing of a gate in an image intensifier 41 with a gate function. A detection means 50, such as a CCD camera, is connected to the image intensifier 41 with a gate function, and the gate is opened or closed based on the gate signal S2. Fluorescence signal Lt that has been received while the gate is open is amplified, and fluorescent spectrum image Ls, the contrast of which has been improved, is received by the detection means 50, such as a CCD. Further, the fluorescent spectrum image Ls is stored as an electrical signal, and detected. For example, when time when the gate controller 42 has received the synchronization signal S1 for the first time is base time t0, if gate signal S2 for instructing the image intensifier 41 with a gate function to open the gate only for time $\Delta t$ is output, the image intensifier 41 with the gate function amplifies the fluorescent signal Lt in a period from time t0 to $\Delta t$, and outputs the amplified signal to the detection means 50, Further, the detection means 50 detects fluorescence Lf in the time range, and outputs the integral value S3 of the intensity of fluorescence to a measurement means 60. This process of detecting the fluorescence intensity S3 is repeated based on the gate signal, and time-resolved fluorescence intensity is output to the measurement means 60.

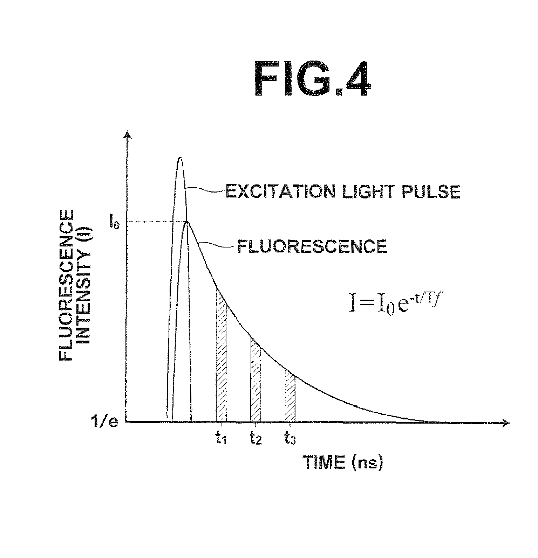

The measurement means 60 is not particularly limited. However, it is easy and desirable to use a computer system, such as a PC, as the detection means 60. The same apparatus may be used as the measurement means 60 and the synchronization control apparatus 43. The measurement means 60 obtains fluorescence intensity time distribution by obtaining a fluorescence decay curve based on the time-resolved fluorescence intensity S3. In the fluorescence decay curve, a difference between time tmax and time t0 is calculated as fluorescence lifetime Tf. The time tmax is time when the fluorescence intensity becomes 1/e, which is substantially zero, and the time t0 is time when emission of fluorescence starts. Figure 4 is a diagram illustrating fluorescence intensity time distribution obtained based on time-resolved fluorescence intensity and fluorescence lifetime Tf obtained from the fluorescence decay curve. In Figure 4, fluorescence intensity I when time period t has passed from time t0 and fluorescence intensity $I_0$ at time t0 have a relationship represented by the following formula:

$$I = I_0 e^{-t/Tf}.$$

In the analysis means 60, S3 is the fluorescence intensity I at time t.

Next, the measurement means 60 measures oxygen concentration at a predetermined position in the substance D to be measured based on the obtained fluorescence lifetime Tf. It is known that the fluorescence lifetime of autofluorescent material changes depending on oxygen concentration in the vicinity of the autofluorescent material. When the autofluorescent material emits fluorescence, quenching occurs by collision with oxygen atoms present around the autofluorescent material. Therefore, when the oxygen concentration around the autofluorescent material becomes lower, the fluorescence lifetime becomes longer.

Therefore, if the fluorescence lifetime of substance D to be measured in normal condition is obtained in advance, it is possible to measure a change in oxygen concentration at a predetermined position in the substance D to be measured based on the fluorescence lifetime of auto fluorescent material at the predetermined position. Further, if a rate of change in fluorescence lifetime with respect to oxygen concentration is obtained in advance, it is possible to measure a specific oxygen concentration values.

In the oxygen concentration measurement apparatus 1, the stage 90 is movable in the directions of x, y and z (in-plane direction and depth direction of an excitation light illumination plane). Therefore, two-dimensional or three-dimensional measurement of oxygen concentration is possible at predetermined plural positions of the substance D to be measured.

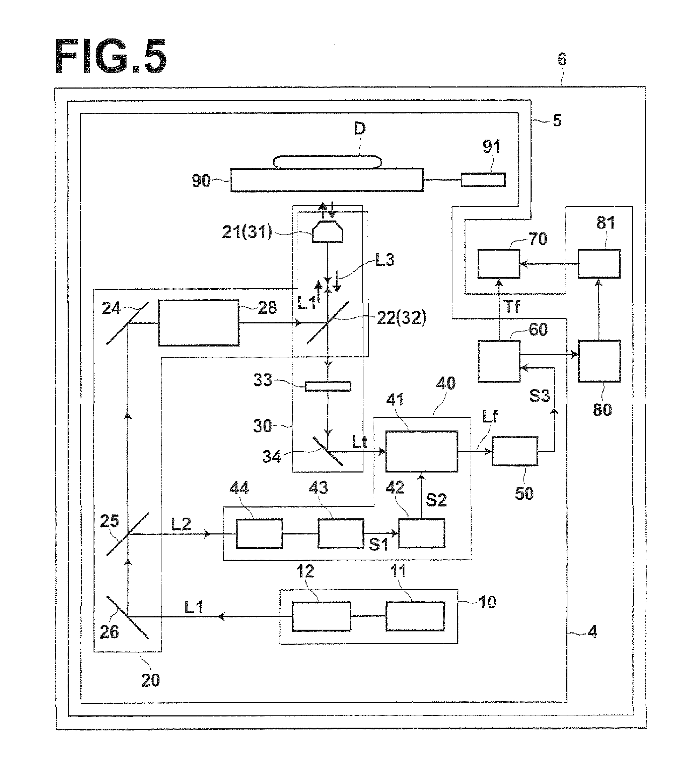

Further, as illustrated in Figure 5, an oxygen concentration measurement apparatus 4 may use a wide field type microscope method, in which a stage 90 is movable in x, y and z directions, and a telescope 28 is provided in the excitation light illumination means 20. In the wide field type microscope method, detection can be performed at once for a wide illumination range. When the stage 90 is scanned in x and y directions, plural xy plane regions are detected. When the detected xy plane regions are combined, it is possible to generate an image of a wider region.

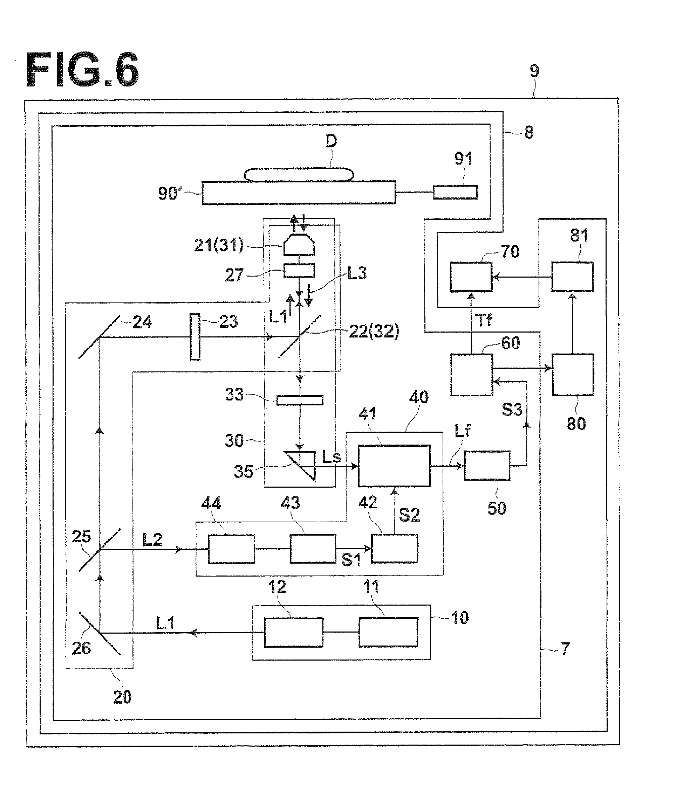

Alternatively, as illustrated in Figure 6, the stage 90' may be movable only in z direction. Further, a confocal optical system in which a scan optical system 27, such as a scan head, scans in x and y directions, may be adopted. In this case, the scan optical system 27 may scan in x, y and z directions.

Examples of the scan optical system 27 are a galvanomirror, a polygon mirror, a resonant mirror, and the like.

When the oxygen concentration measurement apparatus is structured in such a manner, there is a high possibility that plural kinds of fluorescence are excited by excitation light L1. Therefore, as in an oxygen concentration measurement apparatus 7 illustrated in Figure 6, it is desirable that a spectral means 35 is provided. The spectral means 35 obtains a spectrum of fluorescence Lf received by the light receiving means 30, and outputs fluorescent spectrum Ls of the fluorescence Lf to the time-resolving means 40. Since the fluorescent spectrum of the fluorescence Lf is obtained, and the fluorescent spectrum Ls is time-resolved, it is possible to more accurately divide the plural kinds of fluorescence from each other, and to accurately measure the lifetime of fluorescence of a predetermined wavelength. The spectral means 35 is not particularly limited as long as a spectrum of the fluorescence Lf is obtainable, and for examples, a diffraction grating, a prism or the like may be used.

The structure including the scan optical system, as illustrated in Figure 6, is especially desirable when multiphoton excitation that provides localized excitation is performed.

A method for analyzing a low oxygen region of the substance D to be measured by measuring oxygen concentration at plural positions will be described later.

The oxygen concentration measurement apparatuses according to the embodiments of the present invention are structured as described above.

Next, an example of operations of an oxygen concentration measurement apparatus 1 structured as described above will be described.

First, substance D to be measured is placed on the stage 90, specifically in contact with a surface of the stage 90, and the position of the stage 90 is adjusted by the position adjustment means 91 so that a predetermined position is illuminated with excitation light L1.

The excitation light generation means 10 outputs excitation light L1 including a wavelength that can excite fluorescent material P. The excitation light L1 enters the excitation light illumination means 20. Signal light L2 for synchronization is separated, by the beam splitter 25, from the excitation light L1 that has entered the excitation light illumination means 20, and the excitation light L1 is condensed by the objective lens (optical system) 21. Further, a predetermined position in the substance D to be measured is illuminated with the excitation light L1. When the substance D to be measured is illuminated with the excitation light L1, fluorescent material P is excited, and fluorescence Lf is emitted from the fluorescent material P. The objective lens 31 of the light receiving means 30 receives light L3 output from the substance D to be measured. The light L3 includes noise light other than the fluorescence Lf. However, light other than the fluorescence Lf is substantially removed by passing the light L3 through the filter 34.

The light L3 that includes substantially only fluorescence enters the time-resolving means 40, and predetermined fluorescence Lf is extracted, and time-resolved. Further, the detection means 50 detects fluorescence intensity S3 at each time, and the detected fluorescence intensity S3 is output to the measurement means 60 as an electrical signal. The measurement means 60 obtains a fluorescence intensity - time curve based on the time-resolved fluorescence intensity. Further, a difference between time tmax and time t0 is calculated as fluorescence lifetime Tf in the fluorescence intensity - time curve. The time tmax is time when the fluorescence intensity substantially becomes 1/e. Further, the oxygen concentration of the substance D to be measured is measured based on the fluorescence lifetime Tf.

As described above, in the oxygen concentration measurement method and the oxygen concentration measurement apparatus 1 according to embodiments of the present invention, when oxygen concentration of living matter is measured based on fluorescence lifetime Tf of autofluorescent material P, highly accurate measurement is possible by adopting, as the autofluorescent material P, a fluorescent material the fluorescence lifetime of which is longer than or equal to 4.8 nanoseconds. Since the fluorescence lifetime is unique to a material, the oxygen concentration measurement method and the oxygen concentration measurement apparatus 1 according to the present embodiment can highly accurately measure the oxygen concentration of living matter without being influenced by the intensity of excitation light, the concentration of fluorescent material P, and fluorescence from unwanted material.

Further, in the embodiments of the present invention, the fluorescence lifetime of autofluorescent material is a target of measurement. Therefore, it is not necessary to administer a drug or the like to living matter. Hence, measurement of oxygen concentration is possible without greatly damaging the living matter.

The advantageous effects of the oxygen concentration measurement apparatus 1, as described above, are achievable also by oxygen concentration measurement apparatuses in other embodiments that will be described later.

"Second Embodiment of Oxygen Concentration Measurement apparatus (Fiber Probe)"

Next, an oxygen concentration measurement apparatus according to another embodiments of the present invention will be described. The oxygen concentration measurement apparatus 1 in the first embodiment is a microscope. However, an oxygen concentration measurement apparatus 10 in the second embodiment is a fiber probe. In the descriptions of the present embodiment and the drawings, the same signs will be assigned to elements that have substantially the same functions as those of the first embodiment, and explanations will be omitted.

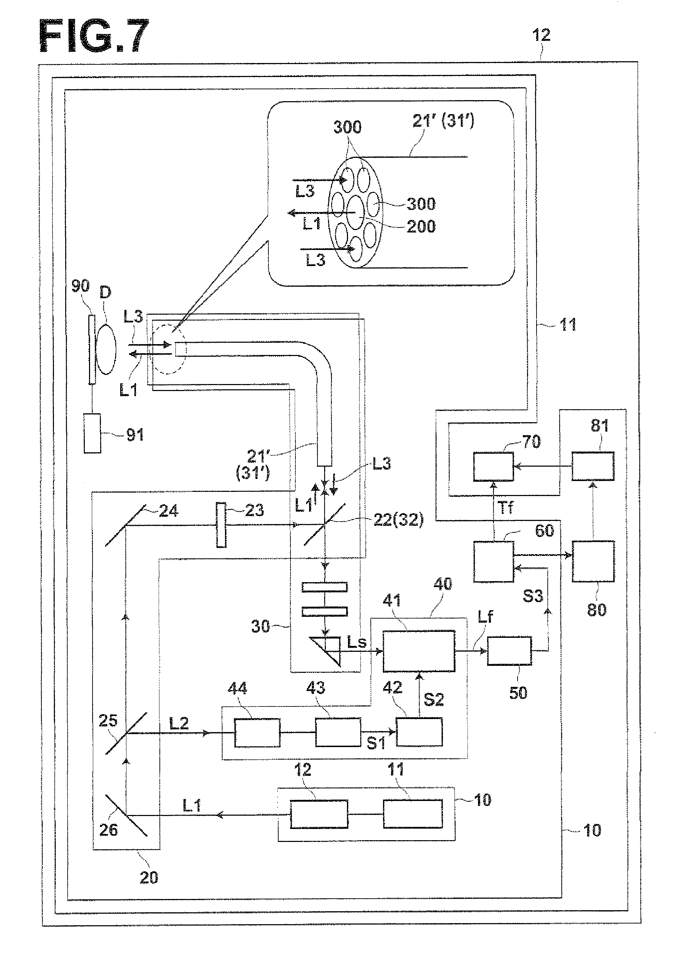

As illustrated in Figure 7, the structure of the oxygen concentration measurement apparatus 10 is similar to the structure of Embodiment 1, except that the objective lens 21 (31) in the first embodiment is replaced by a bundle fiber 21′ (31′) in the second embodiment.

In the bundle fiber 21′ (31′), an optical fiber 200 for illumination is arranged substantially at the center. The optical fiber 200 for illumination guides the pulsed excitation L1 toward a central part, and illuminates a predetermined position in the substance D to be measured with the excitation light L1. Further, plural optical fibers 300 for receiving light are arranged so as to surround the outer surface of the optical fiber 200 for illumination. The optical fibers 300 for receiving light receive light L3 including fluorescence Lf emitted by illumination with the excitation light L1, from autofluorescent material P contained in the substance D to be measured, the fluorescence lifetime of the autofluorescent material P being longer than or equal to 1 nanosecond. Further, the optical fibers 300 for receiving light guide the light L3 to the time-resolving means 40. In Figure 7, seven optical fibers 300 for receiving light are illustrated. However, it is not necessary that the number of the optical fibers 300 for receiving light is seven. At least one optical fiber for illumination and at least one optical fiber for receiving light should be provided, and the number of fibers and the fiber diameters may be appropriately selected based on receivable fluorescence intensity or the like. An objective lens is attached to the leading end of the optical fiber for illumination.

The bundle fiber 21′ (31′) is produced by fixing the optical fiber 200 for illumination and the optical fiber or fibers 300 for receiving light together by using an adhesive or the like so that they are arranged at predetermined positions. It is desirable to use an additive having a heat resistance temperature appropriate for the excitation L1 used in measurement. When a heat resistance temperature exceeding 300°C is required, an inorganic heat-resistant additive is used. Further, the fixed bundle fiber may be used as a fiber probe (not illustrated) by providing the bundle fiber in a long and substantially cylindrical sheath to be inserted into body cavity.

The optical fiber 200 for illumination and the optical fibers 300 for receiving light are not particularly limited. However, it is desirable that the material of the optical fiber 200 for illumination and the optical fibers 300 for receiving light has excellent transmittance of the excitation light L1, and is not damaged by the excitation light L1. Especially, when the excitation light L1 is ultraviolet light or short wavelength light close to the ultraviolet light, the light energy of the excitation light L1 is high. Therefore, in that case, it is desirable to use quartz-based optical fibers.

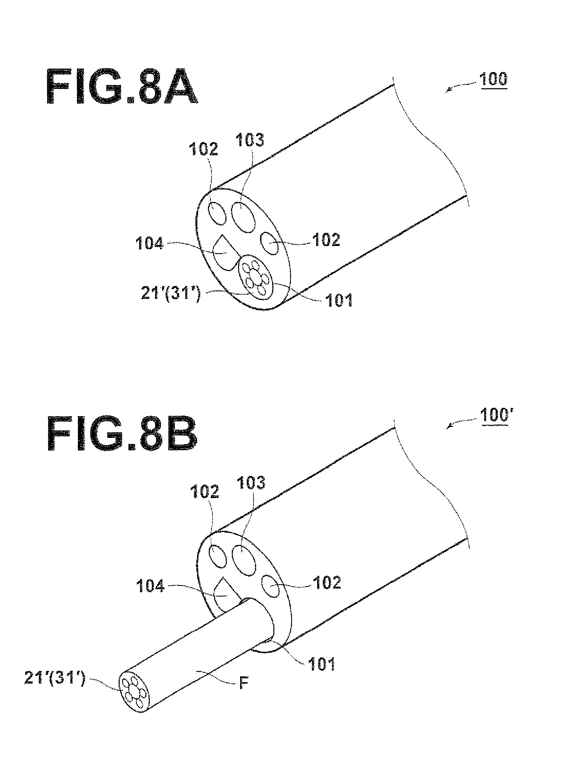

In the second embodiments, it is possible to illuminate the substance D to be measured with the excitation light L1 and to receive light L3 including fluorescence Lf by using fiber probe F. Therefore, measurement in a living body, such as measurement in vivo, is possible. Further, a predetermined position in the substance D to be measured may be determined by an examiner (a user or doctor who performs measurement) by directly moving the probe during examination. For example, as illustrated in Figures 8A and 8B, the bundle fiber 21′ (31′) may be provided in a forceps channel 101 in an endoscope 100. Alternatively, the fiber probe F may be inserted into the forceps channel 101 in the endoscope 100. In this case, as illustrated in Figures 8A and 8B, the endoscope 100 includes an illumination unit 102, an imaging unit 103, and an air/water supply nozzle 104. The illumination unit 102 illuminates a predetermined position in the body cavity with illumination light. The imaging unit 103 images reflection light L4 reflected at a predetermined position. The illumination unit 102 is connected to a light source that can illuminate a predetermined position. The imaging unit 103 is connected to a monitor (not illustrated) or the like that can make the examiner recognize or check the predetermined position.

In the second embodiment, it is possible to directly measure the oxygen concentration of tissue of an animal and a human. For example, the oxygen concentration measurement apparatus according to the second embodiment is appropriate for examination of human digestive organs, tracheas and the like.

"Low Oxygen Region Detection Apparatus (Detection Apparatus)"

With reference to Figures 1, 5 and 6, low oxygen region detection apparatuses (detection apparatuses) 2, 5 and 8 of the present embodiment will be described.

As already described, in the oxygen concentrations measurement apparatus 1 (4, 7), the stage 90 (90′) is movable in xy direction (in-plane direction of the excitation light illumination plane) and z direction (the direction of the optical axis). Therefore, it is possible to measure oxygen concentration at predetermined plural positions in the substance D to be measured. Therefore, for example, when the predetermined plural positions are evenly set in the xy plane of the substance D to be measured, it is possible to obtain in-plane oxygen concentration distribution of the substance D to be measured. Further, when multi-photon excitation, such as two photon fluorescence, is used, it is possible to detect fluorescence at a deep part of the substance to be measured. In that case, it is possible to obtain three-dimensional oxygen concentration distribution by setting measurement positions also in the z direction of the substance D to be measured.

When the oxygen concentration measurement apparatus is a microscope type, as illustrated in Figures 1, 5 and 6, a wide field method that obtains information about xy plane at once, as illustrated in Figures 1 and 5, may be adopted. Alternatively, a confocal method as illustrated in Figure 6 may be adopted.

Therefore, when a low oxygen region detection means 80 that can detect oxygen concentration distribution of the substance D to be measured is provided for the oxygen concentration measurement apparatuses 1, 4 and 7, it is possible to use them, for example, as low oxygen region detection apparatuses 2 (5, 8) for detecting a low oxygen region.

As the low oxygen region detection means, an array detector, such as a CCD and a cooling CCD, may be used.

When the low oxygen region detection apparatus 2 (2′) further includes a display means 81 that generates and displays an image of a detection result obtained by the low oxygen region detection means 80, it is possible to clearly recognize in-plane oxygen concentration distribution and a low oxygen region. As the method for generating the image, a conventional method may be used. For example, a method of displaying in-plane oxygen distribution by using different colors, a method of three-dimensionally displaying in-plane concentration distribution, and the like may be used. When plural images are obtained, they may be combined and displayed.

Further, when the oxygen concentration measurement apparatus 10 of fiber probe type, as illustrated in Figure 7, is used, an examiner can directly move the probe to measure oxygen concentration, at an arbitrary region. Therefore, when a low oxygen region detection means 80 that can detect oxygen concentration distribution of the substance D to be measured is provided in a manner similar to the oxygen concentration measurement apparatus 1, the oxygen concentration measurement apparatus 10 can function as a low oxygen region detection apparatus 11.

In this embodiment, an MEMS (micro-electro mechanical system) scanner, which is attached to the leading end of a fiber probe during use, or the like may be used as the low oxygen region detection means 80. Further, it is possible to perform image detection in a desirable manner by providing an operation unit 91 for the stage 90 on which the substance to be measured is placed.

The low oxygen region detection apparatus 2 (2′) of the present embodiment includes the oxygen concentration measurement apparatus of the above embodiment. Therefore, the low oxygen region detection apparatus 2 (2′) can achieve an advantageous effect similar to the oxygen concentration measurement apparatus 1 (1′).

"living Matter Analysis Apparatus"

With reference to Figures 1, 5, 6 and 7, living matter analysis apparatus 3 (6, 9 and 12) of the present embodiment will be described. The living matter analysis apparatus 3 (6, 9 and 12) is structured by providing an analysis means 70 for the low oxygen region detection apparatus 2 (5, 8 and 11). The analysis means 70 identifies, based on measured oxygen concentration, a pathological condition of the substance D to be measured.

It is known that when a living tissue becomes abnormal, the oxygen concentration in the tissue changes. For example, when a tissue is cancerized (malignant tumor), or when a disease, such as a liver disease, is present, the value of oxygen concentration becomes low. Therefore, it is possible to identify a pathological condition, such as whether a malignant tumor condition is present, and the degree of malignant condition at a predetermined position or region in the substance D to be measured by using the oxygen concentration measurement apparatus 1 (4, 7 and 10) or the low oxygen region detection apparatus 2 (5, 8 and 11) .

In the structure of the present embodiment, it is possible to obtain the in-plane oxygen concentration distribution of the substance D to be measured. Therefore, it is possible to identify an abnormal range of the pathological condition with respect to the in-plane direction of the substance D to be measured. Further, it is possible to identify the degree of the abnormal condition in the range.

In treatment of malignant tumor (cancer), there are surgical therapy, radiation therapy, and the like. In the surgical therapy, an affected part is extracted by excision. Meanwhile, in the radiation therapy, the affected part is not removed, and cancer cells are killed by irradiating the affected part with radiation. Since the living matter analysis apparatus of the present embodiment can identify a cancerized range, it is possible to accurately remove only a region that needs to be removed in the surgical therapy. In surgery for extracting a cancerous tumor, it is not desirable that cancer cells remain in a patient's body after surgery, because the risk of recurrence increases. However, if tissue is excessively removed because of the fear of recurrence, a burden on the patient increases, and that is not desirable, either. Therefore, only a region that is judged to be necessary to be removed is extracted. After extracting the region, a very small amount of tissue in a region that was in contact with the extracted tumor is removed, and examination is performed on the tissue by using the oxygen concentration measurement apparatus of the present invention to find whether a cancer cell is present in the tissue. If a cancer cell is detected, it is judged that further excision is necessary. If no cancer cell is detected, it is judged that a sufficient region has been removed.

Further, in radiation therapy, it is possible to accurately recognize a region that is necessary to be irradiated with radiation. Therefore, it is possible to minimize a part of the patient's body damaged by radiation. Further, it is known that sensitivity of cancer cells to radiation becomes lower when oxygen concentration is low. In an analysis method that can judge only presence a cancer cell, it is impossible to estimate the degree of sensitivity to radiation. Therefore, it is difficult to adopt an appropriate intensity or dose of radiation in treatment. Hence, it is difficult to achieve a satisfactory treatment result.

As described above, the living matter analysis apparatus of the present embodiment includes the oxygen concentration measurement apparatus that can highly accurately measure oxygen concentration. Therefore, it is possible to obtain highly accurate oxygen concentration information about an affected part, such as a cancerous region, together with the position information about the affected part. Therefore, according to the living matter analysis apparatus of the present embodiment, it is possible to select the type of radiation, the intensity or dose of radiation in an appropriate manner based on the condition of the affected part in radiation therapy. Hence, an excellent treatment result is achievable. Further, in endoscopic submucosal dissection (ESD), it is possible to identify the range and the condition of an affected part. Therefore, it is possible to remove an appropriate region, and to minimize a damage to the patient.

Further, it is necessary to quickly judge whether a cancer should be removed during surgery. Since the oxygen concentration measurement apparatus of the present embodiment can measure oxygen concentration immediately and quickly, as described above, the throughput of the oxygen concentration measurement apparatus is high, and the oxygen concentration measurement apparatus is desirable as an analysis apparatus for determining a treatment policy of cancer.

"Design Modification"

So far, embodiments of the oxygen concentration measurement apparatus, the low oxygen region detection apparatus including the oxygen concentration measurement apparatus and the living matter analysis apparatus according to the present invention have been described. However, the present invention is not limited to the embodiments, and various modifications are possible without deviating from the gist of the present invention.

For example, in the above embodiments, a structure in which a detection means that detects a low oxygen region is provided in the oxygen concentration measurement apparatus has been described. However, it is not necessary that the detection means detects the low oxygen region. A detection means that detects a high oxygen region may be provided.

Identification of the pathological condition of substance D to be measured was described with respect to the structure using the analysis means 70. However, when a relationship between oxygen concentration and the degree of abnormality of the pathological condition is clear, it is possible to identify the pathological condition of living matter by using only the oxygen concentration measurement apparatus and the low oxygen region detection apparatus in the aforementioned embodiments. Therefore, the result of treatment described with respect to the living matter analysis apparatus is achievable also by the oxygen concentration measurement apparatus and the low oxygen region detection apparatus in the above embodiments.

"Examples"

Next, examples of the present invention will be described.

(Examples 1)

Oxygen concentration of a cultured HeLa cell was measured by using a microscope-type oxygen concentration measurement apparatus illustrated in Figure 1. FAD (Flavin Adenine Dinucleotide) was used as an autofluorescent material to be excited, and SHG light of a titanium sapphire laser with a pulse width of 1 picosecond was used as excitation, light. Further, the wavelength of the excitation light was adjusted so that the wavelength became 450 nm, which is the excitation wavelength of FAD. At this time, the pulse energy of excitation light was 25 nJ.

The pH of HeLa cells was adjusted to pH 7.4 in culture medium, and two kinds of HeLa cell with oxygen concentration of 5 mmHg and oxygen concentration of 40 mmHg were prepared. Further, fluorescence lifetimes of the two kinds of HeLa cell were measured for fluorescence wavelength of 520 nm. In living matter, FAD is present in a protein bond state and in a free state. However, FAD in the free state the fluorescence lifetime of which was longer than or equal to 4.8 nanoseconds was measured. The measurement temperature was 37°C. According to the result of measurement, the fluorescence lifetime of FAD was 5.13 nanoseconds for the HeLa cell with oxygen concentration of 5 mmHg, and the fluorescence lifetime of FAD was 5.10 nanoseconds for the HeLa cell with oxygen concentration, of 40 mmHg. As this shows, it was possible to detect, as a difference in fluorescence lifetime, a difference in oxygen concentration between the two kinds of HeLa cells.

(Example 2)

Hematoporphyrin was used as an autofluorescent material to be excited. Further, SHG light of a titanium sapphire laser with a pulse width of 1 picosecond was used as excitation light. Further, the wavelength of the excitation light was adjusted so that the wavelength become 420 nm, which is the excitation wavelength of hematoporphyrin. Other conditions were same as Example 1 (the pulse energy of excitation light was 50nJ). Further, two kinds of HeLa cell with oxygen concentration of 5 mmHg and oxygen concentration of 30 mmHg were prepared. Further, fluorescence lifetimes of the two kinds of HeLa cell were measured for fluorescence wavelength of 615 nm. According to the result of measurement, the fluorescence lifetime of hematoporphyrin was 14.3 nanoseconds for the HeLa cell with oxygen concentration of 5 mmHg, and the fluorescence lifetime of hematoporphyrin was 14.2 nanoseconds for the HeLa cell with oxygen concentration of 30 nmHg. As this shows, it was possible to detect, as a difference in fluorescence lifetime, a difference in oxygen concentration between the two kinds of HeLa cells.

(Example 3).

A laparotomy was performed on a mouse with a cancer in the lower digestive organ that had developed by administration of a drug. The large intestine of the mouse was opened, and a cancerous part in the large intestine was used as a substance to be measured. Further, oxygen concentration was measured by measuring the fluorescence lifetime of FAD in a manner similar to Example 1. In Example 3, oxygen concentration was measured at plural positions, and oxygen concentration of a predetermined region of the opened large intestine was measured. Further, a cooling CCD was used as a low oxygen region detection means, and an image of a low oxygen region was generated and detected. An xy operation unit was provided for the stage, on which the mouse was placed, so that a region around the cancerous part, which was expected to be normal, was measured at the same time, and an image of the region was obtained. A wide field type microscope method was used, and measurement was performed on a small region on an xy plane at the same time. Further, the xy stage was operated to obtain plural images of small regions on the xyplane. Accordingly, measurement on a wide range including the cancerous part and the normal region became possible (Figure 5). Further, the plural images were combined. A region to be measured was adjusted to the vicinity of the surface of the substance to be measured by scanning the substance in z direction of the stage. The oxygen concentration of the region expected to be a cancerous part was 3 mmHg in average, while the oxygen concentration of the surrounding region, which was expected to be a normal region, was 29 mmHg in average.

After measurement, the result of measurement was compared with a generated pathological image of the cancerous part. Consequently, it was confirmed that the location of the cancer and the location of the low oxygen region were substantially the same.

Further, similar measurement was performed by two photon excitation by using, as excitation light, a titanium sapphire laser with a pulse width of 200 femtoseconds (wavelength is 840 nm, and peak power is 2 kW), and the fluorescence lifetime of hematoporphyrin was measured. An operation unit for z direction was provided for the stage, on which the mouse was placed, so that detection with respect to the depth direction of the substance to be measured became possible (Figure 6). In this example, a laser scan microscope method was used, and the substance to be measured was scanned in xy directions. First, the laser was focused on the surface of the substance to be measured to obtain data about the surface. Further, the operation unit for z direction of the stage was operated by a pitch of 0.05 mm, and data for xy direction was obtained at each depth. Consequently, detection of a low oxygen region was performed on a region within the depth of 0.2 mm from the surface. The oxygen concentration of a region expected to be a cancerous region was 5 mmHg in average, while the oxygen concentration of a surrounding region, which was expected to be a normal region, was 35 mmHg. It was possible to obtain a three-dimensional image of the low oxygen region of the substance to be measured based on the obtained data.

(Example 4)

A fiber probe type oxygen concentration measurement apparatus illustrated in Figure 7 was used, and low oxygen region detection was performed on a substance to be measured in a manner similar to Example 3. A laparotomy was performed on a mouse, and the large intestine of the mouse was opened. A cancerous part in the large intestine was used as a substance to be measured. Measurement was performed by placing the leading end of the fiber probe close to a region to be measured. In this example, the fiber probe was fixed, and an xy operation unit was provided for the stage, on which the mouse was placed. Accordingly, the affected part of the mouse and a region in the vicinity of the affected part were measured (Figure 7). Consequently, in a manner similar to Example 3, it was confirmed that the location of the detected cancer and the location of the low oxygen region were substantially the same.

"Industrial Applicability"

The oxygen concentration measurement method, the low oxygen region detection method, and the living matter analysis method of the present invention and the apparatuses for carrying out the methods are desirably used in diagnosis as to whether a cell is a normal cell or an abnormal cell (cancer cell or the like) and in separation of the normal cell and the abnormal cell from each other.

**Claims**

1. A measurement method comprising the steps of:

   generating pulsed excitation light (L1) including a wavelength that can excite a fluorescent material contained in living matter, the fluorescence lifetime (Tf) of the fluorescent material being longer than or equal to 4.8 nanoseconds;
   illuminating a predetermined position in the living matter with the pulsed excitation light (L1);
   receiving light including fluorescence (Lf) emitted from the fluorescent material excited by illumination with the pulsed excitation light (L1);
   calculating the lifetime (Tf) of the fluorescence (Lf) included in the received light by time-resolving the intensity of the fluorescence (Lf); and
   measuring the oxygen concentration of the living matter based on the lifetime (Tf).

2. A measurement method, as defined in Claim 1, wherein the fluorescent material is at least one kind of fluorescent material selected from the group consisting of porphyrins, flavin enzymes, collagen, and elastic.

3. A measurement method, as defined in Claim 1 or 2, wherein the fluorescence lifetime (Tf) of the fluorescent material is longer than or equal to 13.3 nanoseconds.

4. A measurement method, as defined in any one of Claims 1 to 3, wherein the lifetime (Tf) of the fluorescence (Lf) included in the received light is calculated for each wavelength by wavelength-resolving the fluorescence (Lf) to obtain fluorescence spectrum (Ls) of the fluorescence (Lf) and by time-resolving, based on the fluorescence spectrum (Ls), the intensity of the fluorescence (Lf) for the respective wavelength.

5. A measurement method, as defined in any one of Claims 1 to 4, wherein the fluorescence (Lf) is excited by multi-photon excitation.

6. A measurement method, as defined in any one of Claims 1 to 5, wherein the predetermined position is a plurality of positions.

7. A detection method, wherein a low oxygen region in the living matter is detected based on oxygen concentrations obtained at the plurality of positions by the measurement method, as defined in Claim 6.

8. A detection method, as defined in Claim 7, wherein the low oxygen region in the living matter is detected by generating and displaying an image of the low oxygen region.

9. A living matter analysis method, wherein the pathological condition of the living matter is identified based on the oxygen concentration measured by using the measurement method, as defined in any one of Claims 1 to 6.

10. A living matter analysis method comprising the steps of:

    detecting the low oxygen region by using the detection method, as defined in Claim 7 or 8; and
    identifying the pathological condition of the detected low oxygen region.

11. A living matter analysis method, as defined in Claim 9 or 10, wherein the pathological condition is presence of malignant tumor condition.

**12.** A measurement apparatus (1, 4, 7, 10) comprising:

an excitation light generation means (10) that generates pulsed excitation light (L1) including a wavelength that can excite a fluorescent material contained in living matter, the fluorescence lifetime (Tf) of the fluorescent material being longer than or equal to 4.8 nanoseconds;

an excitation light illumination means (20) that illuminates a predetermined position in the living matter with the pulsed excitation light (L1);

a light receiving means (30) that receives light including fluorescence (Lf) emitted from the fluorescent material excited by illumination with the pulsed excitation light (L1);

a time-resolving means (40) that time-resolves the fluorescence (Lf) synchronously with illumination with the pulsed excitation light (L1);

a detection means (50) that detects the time-resolved fluorescence (Lf); and

a measurement means that calculates the lifetime (Tf) of the fluorescence (Lf) emitted from the fluorescent material based on the time-resolved fluorescence (Lf) detected by the detection means, and measures the oxygen concentration of the living matter based on the lifetime (Tf) .

**13.** A measurement apparatus (1, 4, 7, 10), as defined in Claim 12, further comprising:

a spectral means that separates the fluorescence (Lf) included in the light received by the light receiving means (30) to obtain wavelength-resolved fluorescence (Lf), and outputs the wavelength-resolved fluorescence (Lf) to the time-resolving means (40).

**14.** A measurement apparatus (1, 4, 7, 10), as defined in Claim 12 or 13, wherein the fluorescent material is at least one kind of fluorescent material selected from the group consisting of porphyrins, flavin enzymes, collagen, and elastin.

**15.** A measurement apparatus (1, 4, 7, 10), as defined in any one of Claims 12 to 14, wherein the fluorescence lifetime (Tf) of the fluorescent material is longer than or equal to 13.3 nanoseconds.

**16.** A measurement apparatus (1, 4, 7, 10), as defined in any one of Claims 12 to 15, wherein the predetermined position is a plurality of positions.

**17.** A measurement apparatus (1, 4, 7, 10), as defined in any one of Claims 12 to 16, wherein the fluorescence (Lf) is excited by multi-photon excitation.

**18.** A measurement apparatus (1, 4, 7, 10) as defined in Claim 17, wherein the excitation light generation means includes a laser (11) that generates pulses with a pulse width in the range of from a femtosecond to hundreds of picoseconds.

**19.** A measurement apparatus (1, 4, 7, 10), as defined in any one of Claims 12 to 18, further comprising:

a stage (90, 90′) that keeps the living matter in contact with a surface of the stage (90, 90′) and which is movable in three-dimensional directions so that an arbitrary position in the living matter is illuminated with the pulsed excitation light (L1); and

a position adjustment means (91, 91′) that moves the stage (90, 90′) to an arbitrary position in three-dimensional directions,

wherein the excitation light illumination means (20) includes an optical system (21, 31) that receives the pulsed excitation light (L1) and illuminates the living matter with the pulsed excitation light (L1), and

wherein the light receiving means (30) includes an optical system (21, 31) that receives the light including the fluorescence (Lf) emitted from the fluorescent material excited by illumination with the pulsed excitation light (L1), and that guides the light to the time-resolving means (40).

**20.** A measurement apparatus (1, 4, 7, 10), as defined in any one of Claims 12 to 18, further comprising:
a stage (90, 90′) that keeps the living matter in contact with a surface of the stage (90, 90′) , and which is movable so that an arbitrary position in the living matter, at least in the direction of an optical axis of the pulsed excitation light (L1), is illuminated with the pulsed excitation light (L1) ;
a first position adjustment means (91, 91′) that moves the stage (90, 90′) to an arbitrary position at least in the direction of the optical axis; and
a second position adjustment means (91, 91′) that moves the excitation light illumination means (20) so that an arbitrary position at least in an in-plane direction, which is perpendicular to the optical axis of the pulsed excitation light (L1), in the living matter is illuminated with the pulsed excitation light (L1),

wherein the excitation light illumination means (20) includes an optical system (21, 31) that receives the pulsed excitation light (L1) and illuminates the living matter with the pulsed excitation light (L1), and

wherein the light receiving means (30) includes an optical system (21, 31) that receives the light including the fluorescence (Lf) emitted from the fluorescent material excited by illumination with the pulsed excitation light (L1), and that guides the light to the time-resolving means (40).

21. A measurement apparatus (1, 4, 7, 10), as defined in any one of Claims 12 to 18, further comprising;

a position adjustment means (91, 91') that moves the excitation light illumination means (20) so that an arbitrary position in three-dimensional directions in the living matter is illuminated with the pulsed excitation light (L1),

wherein the excitation light illumination means (20) includes an optical system (21, 31) that receives the pulsed excitation light (L1) and illuminates the living matter with the pulsed excitation light (L1), and

wherein the light receiving means (30) includes an optical system (21, 31) that receives the light including the fluorescence (Lf) emitted from the fluorescent material excited by illumination with the pulsed excitation light (L1), and that guides the light to the time-resolving means (40).

22. A measurement apparatus (1, 4, 7, 10), as defined in any one of Claims 12 to 21, wherein the excitation light illumination means (20) includes at least one optical fiber (200) for illumination that illuminates the living matter with the pulsed excitation light (L1), and

wherein the light receiving means (30) includes at least one optical fiber (300) for receiving light that receives the light including the fluorescence (Lf) emitted from the fluorescent material excited by illumination with the excitation light (L1) and that guides the fluorescence (Lf) to the time-resolving means (40).

23. Measurement apparatus (1, 4, 7, 10), as defined in Claim 22, wherein the at least one optical fiber (200) for illumination and the at least one optical fiber (300) for receiving light form a bundle fiber (21', 31').

24. A measurement apparatus (1, 4, 7, 10), as defined in Claim 23, wherein the bundle fiber (21', 31') is formed by bundling an optical fiber (200) for illumination and a plurality of optical fibers (300) for receiving light together in such a manner that the outer surface of the optical fiber (200) for illumination arranged substantially at the center of the bundle fiber (21', 31') is surrounded by the plurality of optical fibers (300) for receiving light.

25. A measurement apparatus (1, 4, 7, 10), as defined in Claim 23 or 24, wherein the bundle fiber (21', 31') is a fiber probe that is provided in a substantially-cylindrical long sheath to be inserted into body cavity.

26. A measurement apparatus (1, 4, 7, 10), as defined in Claim 23 or 24, wherein the bundle fiber (21', 31') is provided in a forceps channel of an endoscope (100) that includes an illumination unit (102) for illuminating a predetermined position in body cavity with illumination light, an imaging unit (103) that images reflection light reflected from the predetermined position, and the forceps channel.

27. A measurement apparatus (1, 4, 7, 10), as defined in Claim 25, wherein the fiber probe (21', 31') is provided in a forceps channel of an endoscope (100) that includes an illumination unit (102) for illuminating a predetermined position in body cavity with illumination light, an imaging unit (103) that images reflection light reflected from the predetermined position, and the forceps channel in such a manner that the fiber probe (21', 31') projects from an opening of the forceps channel on the predetermined position side.

28. A detection apparatus (2, 5, 8, 11) comprising :

a measurement apparatus (1, 4, 7, 10), as defined in any one of Claims 12 to 27; and

a low oxygen region detection means that detects a low oxygen region in the living matter based on oxygen concentrations of a plurality of positions measured by the measurement apparatus (1, 4, 7, 10).

29. A detection apparatus (2, 5, 8, 11), as defined in Claim 28, further comprising:

a display device that generates and displays an image of the low oxygen region detected by the low oxygen region detection means (80).

30. A living matter analysis apparatus (3, 6, 9, 12) comprising:

a measurement apparatus (1, 4, 7, 10), as defined in any one of Claims 12 to 29; and
an analysis means that identifies the pathological condition of the living matter based on the oxygen concentration measured by the measurement apparatus (1, 4, 7, 10).

**31.** A living matter analysis apparatus (3, 6, 9, 12), as defined in Claim 30, wherein the pathological condition is presence of malignant tumor condition.

**32.** A living matter analysis apparatus (3, 6, 9, 12), as defined in Claim 30 or 31, further comprising:

a low oxygen region detection means (80) that detects a low oxygen region in the living matter based on the oxygen concentration measured by the measurement apparatus (1, 4, 7, 10) ; and
an analysis means that identifies the pathological condition of the low oxygen region detected by the low oxygen region detection means (80).

**33.** A living matter analysis apparatus (3, 6, 9, 12), as defined in Claim 32, wherein the pathological condition is presence of malignant tumor condition.

# FIG.1

FIG.2

EP 2 365 338 A1

# FIG.3A

# FIG.3B

# FIG.4

FLUORESCENCE INTENSITY (I)

$I_0$

EXCITATION LIGHT PULSE

FLUORESCENCE

$$I = I_0\, e^{-t/Tf}$$

1/e

$t_1$ $t_2$ $t_3$

TIME (ns)

# FIG.5

# FIG.6

EP 2 365 338 A1

# FIG.7

FIG.8A

FIG.8B

Europäisches
Patentamt

European
Patent Office

Office européen
des brevets

# EUROPEAN SEARCH REPORT

**Application Number**

EP 11 15 7639

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | D. SUD ET AL.: "Time-resolved optical imaging provides a molecular snapshot of altered metabolic function in living human cancer cell models", OPTICS EXPRESS, vol. 14, no. 10, 2006, pages 4412-4426, XP002635941, | 1,3,5-9, 12, 15-19,21 | INV. G01N33/84 |
| Y | * page 4414 - page 4418 * <br> * page 4420 - page 4424 * <br> * figures 3,4 * <br> ----- | 12-14, 16-21 | |
| X | SCHWEITZER D ET AL: "[Time-correlated measurement of autofluorescence. A method to detect metabolic changes in the fundus].", DER OPHTHALMOLOGE : ZEITSCHRIFT DER DEUTSCHEN OPHTHALMOLOGISCHEN GESELLSCHAFT OCT 2002 LNKD- PUBMED:12376853, vol. 99, no. 10, October 2002 (2002-10), pages 774-779, XP002635942, ISSN: 0941-293X <br> * page 777, right-hand column * <br> ----- | 1-33 | |
| X | US 2004/073119 A1 (MYCEK MARY-ANN [US] ET AL) 15 April 2004 (2004-04-15) <br> * page 9; table * <br> ----- | 1-33 | TECHNICAL FIELDS SEARCHED (IPC) <br><br> G01N |
| X | WO 2005/019800 A2 (CEDARS SINAI MEDICAL CENTER [US]; JO JAVIER A [US]; MARCU LAURA [US];) 3 March 2005 (2005-03-03) | 1,2,4-6 | |
| Y | * page 3, line 34 - page 4, line 25 * <br> * page 6, line 1 - line 19 * <br> * page 7, line 27 - line 32 * <br> * page 11, line 28 - page 18, line 4 * <br> * page 15, line 7 - line 30 * <br> * page 21, line 4 - page 22, line 6 * <br> * page 27, line 1 - page 29, line 26 * <br> ----- | 7-14, 16-33 | |

-/--

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 365 338 A1

Europäisches
Patentamt
European
Patent Office
Office européen
des brevets

**EUROPEAN SEARCH REPORT**

Application Number

EP 11 15 7639

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YUEZHI LI ET AL: "Quantitative Time-Resolved Fluorescence Spectrum of the Cortical Sarcoma and the Adjacent Normal Tissue", JOURNAL OF FLUORESCENCE, KLUWER ACADEMIC PUBLISHERS-PLENUM PUBLISHERS, NE, vol. 17, no. 6, 23 June 2006 (2006-06-23), pages 643-648, XP019553473, ISSN: 1573-4994 * page 646, right-hand column - page 647, left-hand column, paragraph bridging * * figure 1 * | 7-14, 22-33 | |
| Y | CHUNG A ET AL: "Advanced Optical Imaging Requiring No Contrast Agents-A New Armamentarium for Medicine and Surgery", CURRENT SURGERY, WILIAMS AND WILKINS, BALTIMORE, US, vol. 62, no. 3, 1 May 2005 (2005-05-01), pages 365-370, XP004910715, ISSN: 0149-7944, DOI: DOI:10.1016/J.CURSUR.2004.12.011 * page 366, right-hand column, paragraph 3 - page 369, right-hand column, paragraph 2 * | 12-14, 16-33 | |
| | | | TECHNICAL FIELDS SEARCHED (IPC) |
| X | WO 2008/012785 A2 (UNIV COLLEGE CORK NAT UNIV IE [IE]; PAPKOVSKY DIMITRI [IE]; O'RIORDAN) 31 January 2008 (2008-01-31) | 1-7, 12-17, 19-21,28 | |
| Y | * page 7, line 9 - line 22 * * page 8, line 5 - line 18 * * page 11, line 26 - line 28 * * page 21, line 4 - line 21 * * page 32, line 5 - page 33, line 30 * * examples 3,4,7-11 * * claims 23,30,33-37 * | 12-14, 16-21 | |

-/--

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 11 15 7639

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/115521 A1 (UNIV MICHIGAN [US]; ASHKENAZI SHAI [US]; KOPELMAN RAOUL [US]; WITTE RU) 25 September 2008 (2008-09-25) | 1-3,6-11 | |
| Y | * paragraph [0024] - paragraph [0038] * * paragraph [0053] * | 7-11 | |
| | ----- | | |
| X | KONIG KARSTEN ET AL: "High-resolution multiphoton tomography of human skin with subcellular spatial resolution and picosecond time resolution.", JOURNAL OF BIOMEDICAL OPTICS JUL 2003 LNKD- PUBMED:12880349, vol. 8, no. 3, July 2003 (2003-07), pages 432-439, XP002637697, ISSN: 1083-3668 * table 1 * | 1-33 | |
| | ----- | | |
| A | US 6 272 376 B1 (MARCU LAURA [US] ET AL) 7 August 2001 (2001-08-07) * the whole document * | 1-33 | |
| | ----- | | TECHNICAL FIELDS SEARCHED (IPC) |
| A | PAUL R J ET AL: "Oxygen concentration and the oxidation-reduction state of yeast: Determination of free/bound NADH and flavins by time-resolved spectroscopy", NATURWISSENSCHAFTEN, vol. 83, no. 1, 1996, pages 32-35, XP002635943, ISSN: 0028-1042 * the whole document * | 1-33 | |
| | ----- | | |

-/--

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

| | Europäisches Patentamt<br>European Patent Office<br>Office européen des brevets | **EUROPEAN SEARCH REPORT** | Application Number<br>EP 11 15 7639 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | URAYAMA P ET AL: "A UV-visible-NIR fluorescence lifetime imaging microscope for laser-based biological sensing with picosecond resolution", APPLIED PHYSICS B: LASERS AND OPTICS, SPRINGER INTERNATIONAL, BERLIN, DE, vol. B76, no. 5, 1 January 2003 (2003-01-01), pages 483-496, XP002289222, ISSN: 0946-2171 * the whole document * | 1-33 | |
| A | GERRITSEN HP ET AL: "Fluorescence lifetime imaging of oxygen in living cells", JOURNAL OF FLUORESCENCE, vol. 7, no. 1, 1 March 1997 (1997-03-01), pages 11-15, XP002635944, DOI: 10.1007/BF02764572 * the whole document * | 1-33 | |
| A | WO 92/13265 A1 (UNIV MARYLAND [US]; NOWACZYK KAZIMIERZ [US]; SZMACINSKI HENRYK [US]; J) 6 August 1992 (1992-08-06) * examples 1-4 * * claims 1,7,27-35 * | 1-33 | **TECHNICAL FIELDS SEARCHED (IPC)** |
| A | VAN MUNSTER ERIK B ET AL: "Fluorescence lifetime imaging microscopy (FLIM)", ADVANCES IN BIOCHEMICAL ENGINEERING, BIOTECHNOLOGY, SPRINGER, BERLIN, DE, vol. 95, 1 January 2005 (2005-01-01), pages 143-175, XP008105148, ISSN: 0724-6145, DOI: DOI:10.1007/B102213 [retrieved on 2005-06-02] * the whole document * | 1-33 | |

The present search report has been drawn up for all claims

| Place of search | Date of completion of the search | Examiner |
|---|---|---|
| The Hague | 18 May 2011 | Chrétien, Eva Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 11 15 7639

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2011

| Patent document cited in search report | | Publication date | Patent family member(s) | | Publication date |
|---|---|---|---|---|---|
| US 2004073119 | A1 | 15-04-2004 | NONE | | |
| WO 2005019800 | A2 | 03-03-2005 | EP | 1659944 A2 | 31-05-2006 |
| | | | JP | 2007502988 T | 15-02-2007 |
| | | | US | 2007197894 A1 | 23-08-2007 |
| WO 2008012785 | A2 | 31-01-2008 | EP | 2043694 A2 | 08-04-2009 |
| | | | US | 2008051646 A1 | 28-02-2008 |
| WO 2008115521 | A1 | 25-09-2008 | US | 2008230717 A1 | 25-09-2008 |
| US 6272376 | B1 | 07-08-2001 | NONE | | |
| WO 9213265 | A1 | 06-08-1992 | EP | 0568596 A1 | 10-11-1993 |
| | | | US | 5485530 A | 16-01-1996 |

EPO FORM P0459

For more details about this annex : see Official Journal of the European Patent Office, No. 12/82

**REFERENCES CITED IN THE DESCRIPTION**

**Patent documents cited in the description**

- JP 2001509589 PCT

- JP 2006282653 A

**Non-patent literature cited in the description**

- **D. SUD et al.** Time-resolved optical imaging provides a molecular snapshot of altered metabolic function in living human cancer cell models. *OPTICS EXPRESS,* 2006, vol. 14 (10), 4412-4426